# EUROPEAN PATENT APPLICATION

(11) **EP 4 342 514 A2**
(43) Date of publication of application: **27.03.2024**
(21) Application number: 24157477.1
(22) Date of filing: 22.08.2018
(51) Int. Cl.: A61M 60/865

(54) **DEVICE FOR COMPRESSING A COMPRESSIBLE PART OF A CATHETER PUMP**

(30) Priority: 23.08.2017 EP 17187609
(62) Divisional of application: 18769592.9
(71) Applicant: ECP Entwicklungsgesellschaft mbH, 52074 Aachen (DE)
(72) Inventor: SCHECKEL, Mario, 14195 Berlin (DE)
(74) Representative: Pfenning, Meinig & Partner mbB

(57) **Abstract**

The invention relates to a system comprising a catheter device with a radially compressible part, a sleeve, and a device for compressing said radially compressible part and introducing the radially compressible part into the sleeve, as well as a method for using such a system.

## Description

The invention relates to a system comprising a catheter pump with a radially compressible part and a device for compressing said compressible part, as well as a method for using said system.

The application can hence be provided, on the one hand, in the minimally invasive medical field, for example for a blood pump for heart assistance, and, on the other hand, also for the use in agitators or drive elements.

The invention can display special advantages by the possible miniaturisation in the medical field. Techniques for introducing fluid pumps, in particular into natural body lumina, are known in detail in prior art. Thus reference is made inter alia to the Seldinger technique for introducing an introducer sheath into a vascular system.

Compressible or expandable catheter pumps, where a radially compressible part, for instance a rotor and a rotor housing, can be transferred into a sleeve or sheath are for instance disclosed in EP2399639 or EP2606920. A sleeve is provided around the catheter and the compressible part is pulled into the sleeve, such that compression is effected as the compressible part enters the sleeve. The sleeve can for instance be a peel-away sheath which facilitates insertion of the catheter pump into a lumen, as described within EP2399639, or any other type of sleeve, for instance a cannula pertaining to the catheter itself.

While such expandable pumps are the current state of the art and allow for minimally invasive introduction into the human body, there are a number of drawbacks regarding the handling of such pumps.

To ensure that the pump functions properly, the compressible part should not be transported and provided in an already compressed state. The time during which the compressible part is compressed should be kept to a minimum, since the flexible parts could start to creep when kept in a compressed state for too long.

According to the prior art, the catheter device is provided in an expanded state. For example, by applying a pulling force on the proximal end of the catheter tube of the catheter device, the compressible part is pulled into the sleeve which is provided on the proximal side of the compressible part. Compression hence is effected at the end of the sleeve and commences on the proximal end of the compressible part. As the compressible part is squeezed into the sleeve, a force with an initially predominantly axial component acts on the rotor and the housing, leading to strong deformation of the compressible components. This procedure causes stress and strain to the compressible part and can lead to kinks in the compressible part.

Furthermore, upon compression, the compressible part often experiences elongation, i.e., in a pump setup where a rotor is located in a housing, both the housing and the rotor can expand axially while being compressed radially. If the rotor and the housing are compressed from one end by being pulled into a sleeve, the axial extension is hindered in the direction of the sleeve. In particular, axial elongation of the rotor in the direction of the sleeve is impeded by the already compressed housing. This can lead to additional strain or kinks in the rotor upon folding.

EP3153190 shows a rotor housing which comprises fewer struts on one end of the housing, to minimize damage to the blood. This, however, increases the risk of the rotor being caught or pinched between the struts upon compression of the housing and the rotor, since the gaps between the struts are larger.

It is therefore advantageous to introduce the compression force on the rotor housing such that the compression force, in particular the initial compression force, is applied more centrally on the rotor and with a larger radial component. By applying the force in a central section of the compressible housing, between the distal end of the housing and the proximal end of the housing, i.e., in an axial section at which the rotor is located within the housing, or close to an axial section at which the rotor is located within the housing, uneven folding or pinching of the rotor between the struts can be avoided. If the force is introduced in such a way, rather than only from one end, the rotor can then elongate and/or shift axially in both the proximal and the distal direction. The rotor can then be smoothly folded around its hub.

In principle, compression of a compressible part of a catheter device could be done using a stent crimper, for instance like the one described in EP 0873731. However, the use of stent crimpers in a catheterization laboratory or a hospital environment is problematic, since stent crimpers are delicate and complicated tools which are hard to clean and sterilize after use.

The aim of the application is therefore to provide an easy way for compressing a compressible part of a catheter pump and inserting said compressible part into a sleeve in a quick, safe and reproducible manner, which is feasible also in a catheterization laboratory.

The above-mentioned problems and requirements are at least in part addressed by a system according to claim 1. Further embodiments which can be advantageous are given by the dependent claims or are disclosed in the description and the figures.

The system according to the application comprises a catheter device with a compressible part. Typically, the compressible part comprises a compressible rotor and a housing for the rotor. The compressible part is positioned near an end of the catheter pump defined as the distal end, which is configured to be inserted into the patient, while a proximal end portion of the catheter remains outside of the patient. A motor connected to the proximal end of the catheter device can then drive a rotor at the distal end of the catheter device by means of a drive shaft. The rotor is for example positioned in the left ventricle of a heart and driven such that a flow of blood in the proximal direction of the catheter device is effected, for instance out of the left ventricle and into the aorta. A downstream tubing can be provided proximally of the rotor, in the section where the aortic valve needs to be passed. The compressible housing of the rotor prevents entanglement of heart tissue with the rotor during operation. The catheter device can further comprise a distal bearing. The distal bearing can comprise an elongated portion for rotatably mounting the drive shaft. It is preferably designed as a flexible bearing. The catheter device can further comprise an atraumatic tip with an elongated flexible portion. The atraumatic tip is preferably designed as a pigtail. In some embodiments, the flexible polymer part which comprises the atraumatic tip or the pigtail tip can comprise the distal bearing.

The system further comprises a sleeve in which the compressible part is to be inserted. The sleeve can either be a peel-away sheath which is then docked to an introducer sheath and is removed and discarded after the compressible part has been transferred to the introducer sheath. It can also be any other type of sleeve, such as a cannula pertaining to the catheter itself or an additionally provided sleeve, depending on the desired use of the catheter pump.

Furthermore, the system comprises a compression pipe. The compression pipe comprises movable fins which are designed to receive the radially compressible part. The movable fins have an open state and a closed state. In the open state, the fins can be positioned around at least a portion of the radially compressible part in the uncompressed state. The opening angle of the compressible fins in an open state is such that the fins can be brought in contact with an axial section of the housing which lies between the distal end and the proximal end of the housing compression, preferably an axial section to which the rotor extends in the expanded state. If the fins are now transferred to the closed state, the radially compressible part is compressed and the compression force is applied at said axial section, wherein the force has a radial component.

In one embodiment the compression pipe comprises between two and ten movable fins. In particular, the compression pipe can comprise three movable fins or four movable fins.

In one embodiment neighboring fins are connected by a flexible membrane or skin to avoid pinching of the compressible part or the downstream tubing.

In the open state, a gap is formed between neighboring fins. As the fins are transferred from the open state to the closed state, the gap size is reduced. In one embodiment, in the closed state, the gap between neighboring fins persists, such that the radially compressible part, the downstream tubing or other parts of the catheter device cannot be pinched or trapped between the fins.

The gap that is formed between neighboring fins can extend axially along the length of the compression pipe. The gap can however also extend with a radial component, such that it runs spirally along the compression pipe. Spirally extending gaps can run around the circumference of the compression pipe and extend radially over for instance between 40 and 100 degrees, in particular 90 degrees. In addition to the gaps, slits can be provided. The slits can be provided as a continuation of the gaps, or they can be provided between the gaps, wherein the gaps extend either axially or spirally. When slits are provided, the compression pipe can be opened further and show smoother compression behavior. By having additional slits and/or by having spirally extending gaps, pinching of parts of the catheter device can be avoided.

In an embodiment, ends of radially displayed slits overlap axially with ends of gaps and/or axially aligned slits.

Preferably, the fins in the closed state confine a cylindrical space. In one embodiment, the diameter of the cylindrical space is at most 1mm smaller or larger than the inner diameter of the sleeve in which the compressible part is to be inserted, preferably at most 0.5 mm, such that the compressible part can easily slide from between the fins in the closed state into the sleeve. In some embodiments, it is desired that the compressible part undergoes further compression as it slides from between the fins into the sleeve.

The compression pipe can further comprise a tube or tubular portion, designed to be positioned around the catheter device. The fins are attached to one end of the tube. When the fins are positioned around the compressible part, there are two possible configurations: the tube can be positioned proximally of the fins and the compressible part, around a portion of the catheter tube, or the tube can be positioned on the distal side of the movable fins and the compressible part, around a portion or all of the distal bearing or the atraumatic tip or pigtail tip.

The tube can comprise a recess for the pigtail, which can be advantageous if positioning of the tube around the atraumatic tip is desired. The pigtail can then be in the curled position, rather than being kept in the elongated and strained position, while the tube is around the elongated portion of the tip, without having to shorten the tube on the distal end, which would complicate the handling of the tube. This is particularly useful when the catheter device is shipped with the compression pipe in place. Allowing the pigtail to curl in the recess, avoids that the tube falls off the distal end of the catheter device.

The system can further comprise a reducer for compressing the movable fins from the open state to the closed state in a smooth and radially isotropic fashion.

The reducer is designed as a tube with a conical section, i.e., a section where an inner wall of the reducer describes a section of a circular conical surface, and a cylindrical section, where the inner wall of the reducer has a constant diameter. In the conical section, the inner radius of the reducer increases as the distance from the cylindrical section increases. An opening angle of the inner wall of the conical section with respect to the inner wall of the cylindrical section can for example be between 6° and 10°.

The reducer can be provided around the catheter device, proximally of the compressible part and distally of the sleeve in which the compressible part is to be inserted. The conical section lies thereby distally of the cylindrical section. Thus, as the reducer is moved in a distal direction with respect to the catheter device and the compression pipe, the compression pipe and the compressible part of the catheter device provided between the fins of the compression pipe slide into a distal opening of the reducer into the conical section of the reducer. The relative movement can for example be effected by pushing the compression part and the reducer together by hand, or by holding on the reducer and pulling in a proximal direction on the proximal end of the catheter tube. The compression pipe either slides into the distal opening of the reducer with the fins first or with the pipe first, depending on the configuration used. As the relative movement continues, and the fins enter the conical section of the reducer, the fins start to be compressed, as the radius of the conical section of the reducer decreases in the direction of relative movement of the compression pipe with respect to the reducer. Therefore, the compressible part provided between the fins is also compressed. Preferably, the compression force mediated by the compressible fins acts on an axial section of the compressible part in which the rotor extends. The compression force then has a radial component, which is advantageous for compressing the rotor without causing kinks. The compression pipe and the compressible part provided in the compression pipe then further slide into the reducer, and into the cylindrical portion of the reducer. When the cylindrical portion is reached, the compression is completed. The fins are now in a fully compressed state and the radially compressible part is compressed to the desired radius. Preferably, a gap between neighboring movable fins remains, also in the compressed state, to avoid pinching of the compressible part or the downstream tubing. This can be further aided by providing membranes, preferably elastic membranes or skins between neighboring movable fins. As the relative movement of the compression pipe and the catheter device with respect to the reducer continues, the compression pipe and the compressible part slide towards a proximal opening of the reducer. Two opposing stops can be provided near the proximal opening. The sleeve in which the compressible part is to be inserted is then provided against the first stop on the proximal side of the proximal opening. The stop limits the movement of the sleeve in the distal direction with respect to the reducer. The compression pipe strikes the second stop, opposing the first stop, on the distal side of the proximal opening, inside the reducer. Thus, the movement of the compression pipe in proximal direction with respect to the reducer is limited. The catheter device can now be pulled out of the compression pipe, for instance by exerting a pulling force on the proximal end of the catheter tube. The compressible part of the catheter device then slides out of the compression pipe, through the proximal opening of the reducer and into the sleeve. The distal bearing and/or the atraumatic tip also slides through the compression pipe and the proximal opening of the reducer. The reducer and the compression pipe then fall of the distal end of the catheter device and can be discarded.

In embodiments envisioning a reducer, the sleeve and/or the compression pipe can each exhibit an additional stop configured to abut against the reducer, preferably against the outside of an end of the reducer. The stop can be positioned in such a way, that a gap is provided on the inside of the reducer at the end of the sleeve and/or at the end of the compression pipe. This way, the catheter device cannot be pinched between the sleeve and the reducer or between the compression pipe and the reducer or between the sleeve and the compression pipe.

Alternatively to what was described so far, the reducer and the compression pipe can be rotated by 180°, such that the reducer is provided distally of the compressible part and the compression pipe proximally of the compressible part. The sleeve can then be provided proximally of the compression pipe or distally of the reducer. The latter option can be advantageous for pumps that are inserted into the right ventricle, which pumps can have a downstream tubing that lies distally of the rotor.

The compression pipe with the compressible part of the catheter device provided between the fins is inserted into the conical portion of reducer, for instance by manually sliding the compression pipe into the reducer. When the compressible fins have reached the cylindrical portion of the reducer, the compression is finished. Preferably, a stop is provided to limit the relative movement of the reducer and the compression pipe, such that the movable fins remain in the cylindrical portion and the compression pipe cannot slide through the reducer. In a next step, the sleeve is provided against a second stop pertaining to the reducer, or directly against the compression pipe which can act as a stop for the sleeve. By holding on to the sleeve and pulling on the catheter tube, the compressible part can slid out of the compression pipe and into the sleeve.

The compression pipe and the reducer are for instance both made of PTFE, PE or POM, such that friction between the compression pipe and the reducer is minimized.

The catheter device can be delivered with the compression pipe and the reducer pre-mounted on the catheter device. The compression pipe and the reducer can be single-use parts which are discarded after use. Additional stops can be provided, for instance in the conical portion of the reducer, to allow pre-mounting the compression pipe in such a way that the compressible fins with the radially compressible part are already provided in the conical portion of the reducer, in an uncompressed state and cannot slide out due to the stops. This way, the compressible part is protected between the reducer and the compression pipe.

In one embodiment, in addition to the catheter device, the reducer and the compression pipe, a disk device, such as a plate or membrane, with a hole is provided. The disk device is provided around the catheter tube, between the reducer and the compression pipe, such that it can slide along the catheter tube. The disk device can have a slit connecting the hole and the outer edge of the disk device, such that the disk device can be clipped on the catheter. In another embodiment, the slit is only connected to the hole but not to the outer edge of the disk device, to prevent the disk device from getting lost. The edges of the disk device, in particular the edges of the slit and of the hole are smooth and/or made of flexible material, such that the catheter cannot be damaged and such that the disk device has good sliding properties on the catheter tube. The radius or radial extension of the disk device is larger than the radius of the cylindrical portion of the reducer. Preferably, the radius or radial extension of the disk device is larger than the radius of a circular plane circumscribed by the front parts of the movable fins in the uncompressed state. The disk device is made of a flexible material, for example PTFE, PE, or Silicone, such that it is capable of elastically yielding as it slides along the catheter device and possesses good sliding properties. As the reducer is moved with respect to the catheter device and the compression pipe, the reducer pushes the disk along the catheter device, the disk provided against the distal end of the reducer. In one embodiment the disk closes the distal opening of the conical portion. When the disk abuts the compressible part, the catheter device, the disk device and the reducer are moved relative to the compression pipe.

If the compression pipe is configured to enter the reducer with the fins first, a part of the reducer near the distal opening abuts the disk as the reducer is moved toward the compression pipe. As the movement continues, the disk device abuts the compressible part and pushes the compressible part between the fins. The disk device can thus help positioning the radially compressible part at a position which is advantageous for the subsequent compression. Continuing the relative movement of the reducer and the compression pipe, the disk device gives in or breaks as it is pushed through or into the conical portion of the reducer, allowing the compression pipe to slide into and through the conical portion of the reducer.

Aspects and embodiments of the system according to the application are exemplified in Figures 1 to 13.
Figure 1 shows a catheter device which is positioned within the left ventricle of a heart;
Figure 2 shows a distal end region of a catheter device;
Figure 3 a and b show the distal end region of the catheter device with a compression tube and a reducer;
Figure 4 shows a compressible part of a catheter device being pulled into a sleeve without compression pipe and reducer;
Figures 5 a and b show the compression pipe from two different perspectives;
Figures 6 a and b show the reducer from two different perspectives;
Figure 7 shows a cut through the reducer;
Figure 8 shows the compression pipe and the reducer without the catheter device;
Figure 9 a and b show the distal end region of the catheter device with a compression tube and a reducer and a disk device;

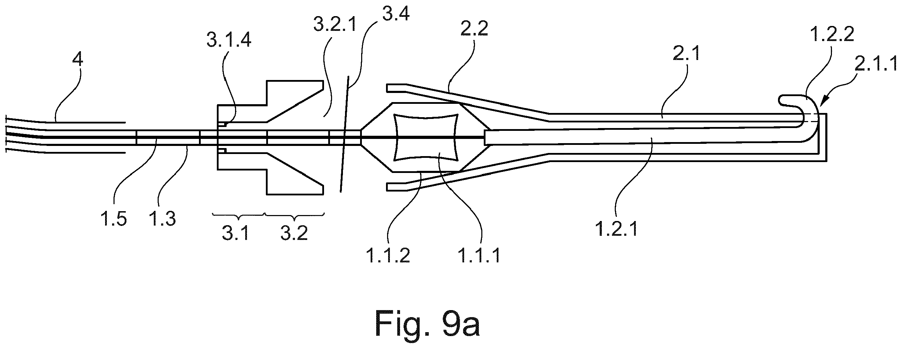

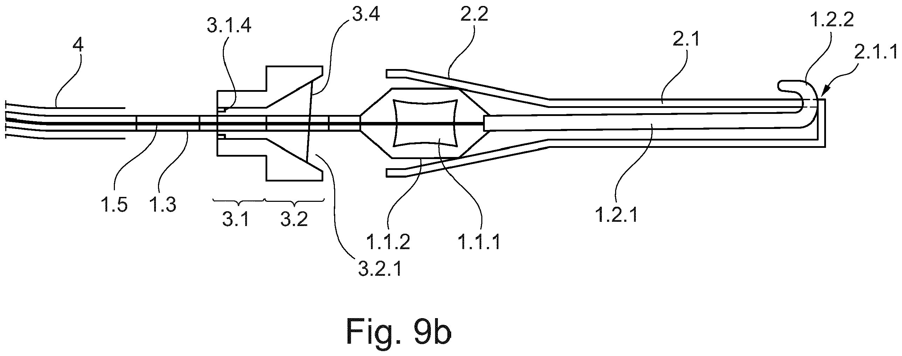

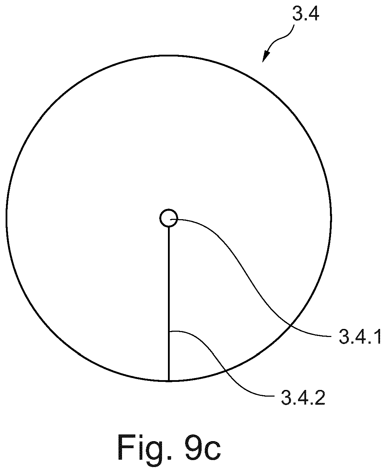

Figure 9 c shows the disk device from Figures 9 a and b;

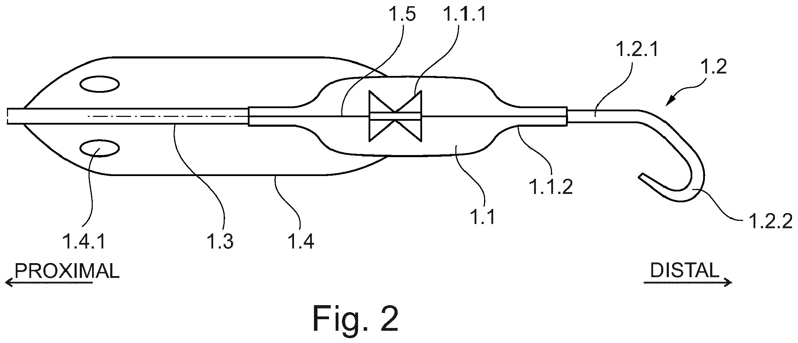

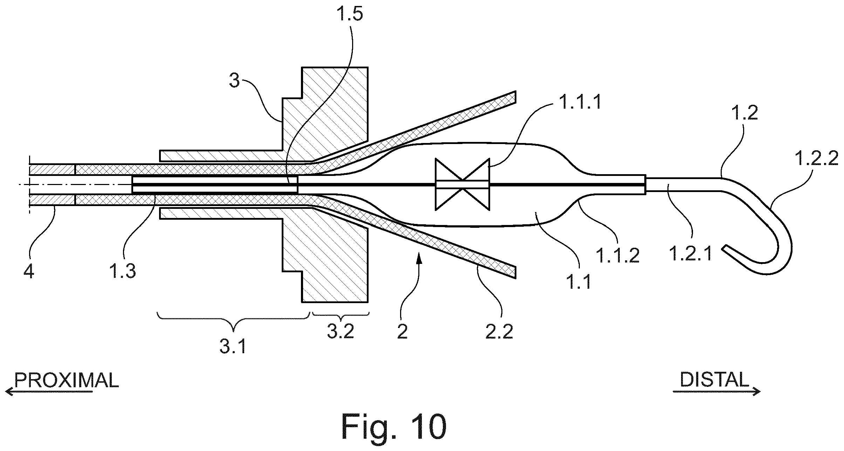

Figure 10 shows a setup as in Figure 2, where the compression tube is provided in an alternative configuration;

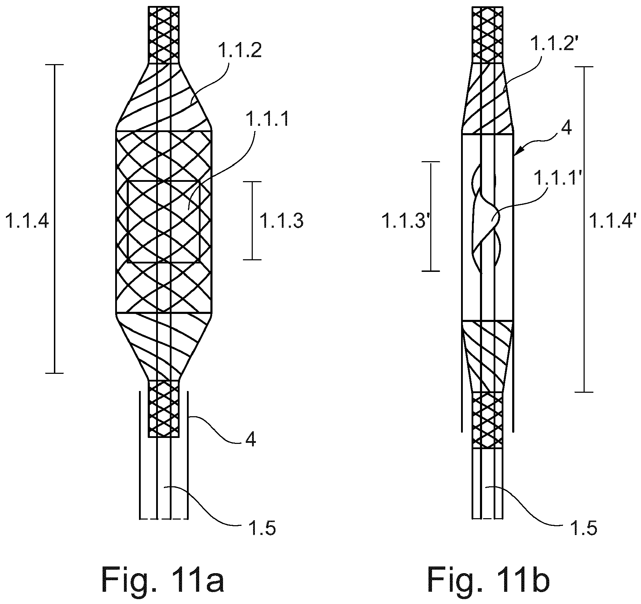

Figures 11 a and b show the rotor and the housing in the expanded state and in the compressed state;

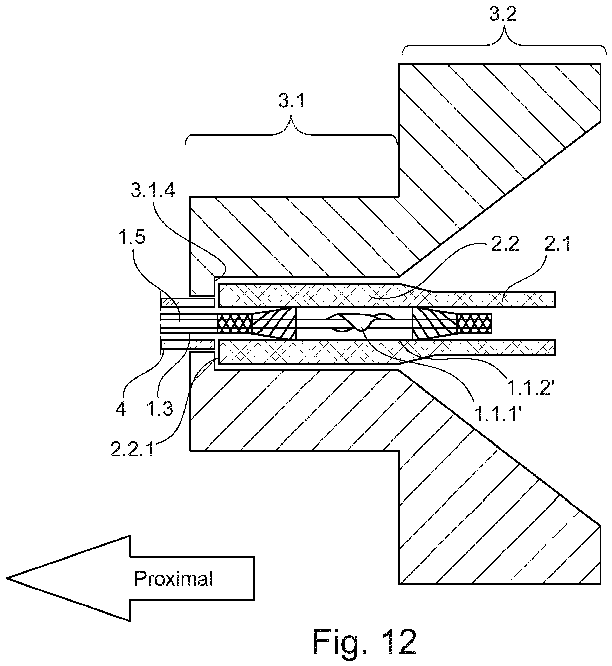

Figure 12 shows the compression tube and the reducer and the catheter device with the compressible part in the compressed state;

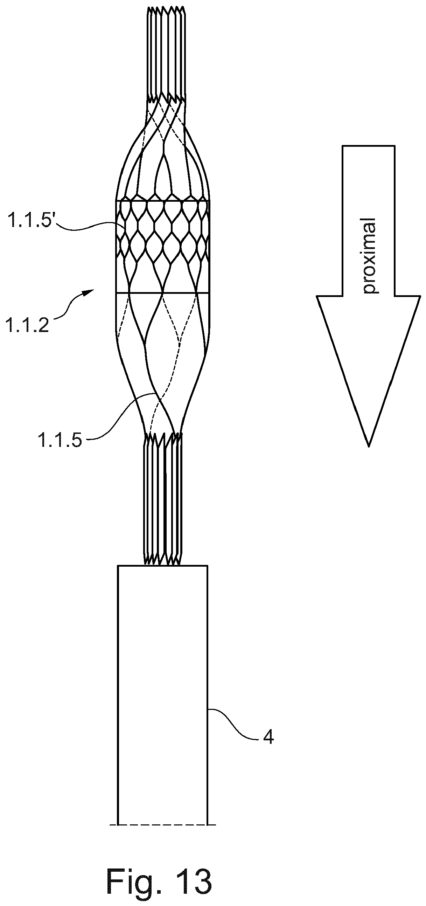

Figure 13 shows a rotor housing with fewer struts near the proximal end;

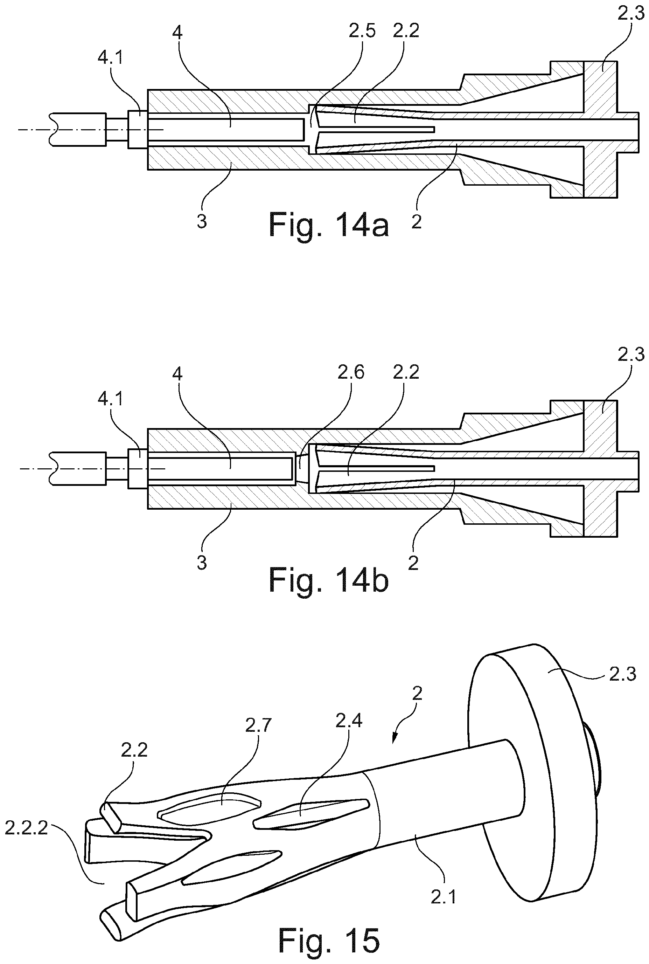

Figures 14a and 14b shows the sleeve and the compression pipe which are introduced into the reducer, the sleeve and the compression pipe having additional stops;
Figure 15 shows an embodiment of the compression pipe with additional slits; and

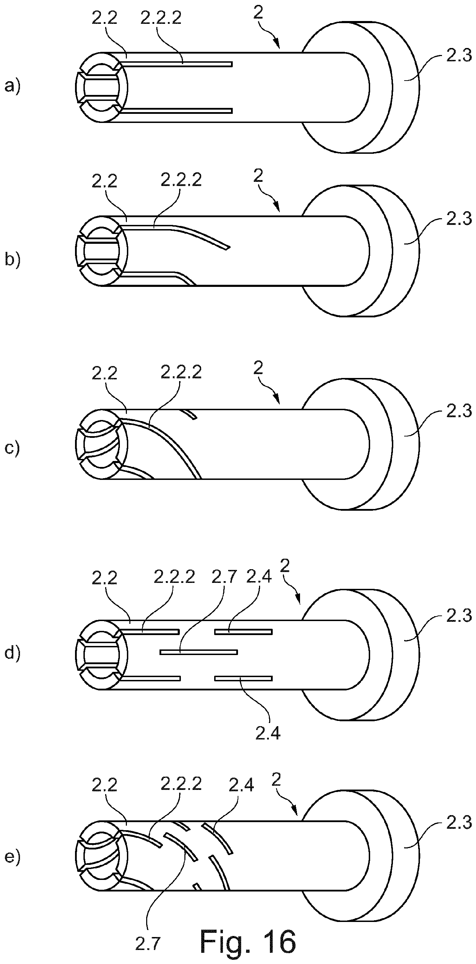

Figures 16a-e show embodiments of the compression pipe exhibiting different configurations for the gaps between the fins.

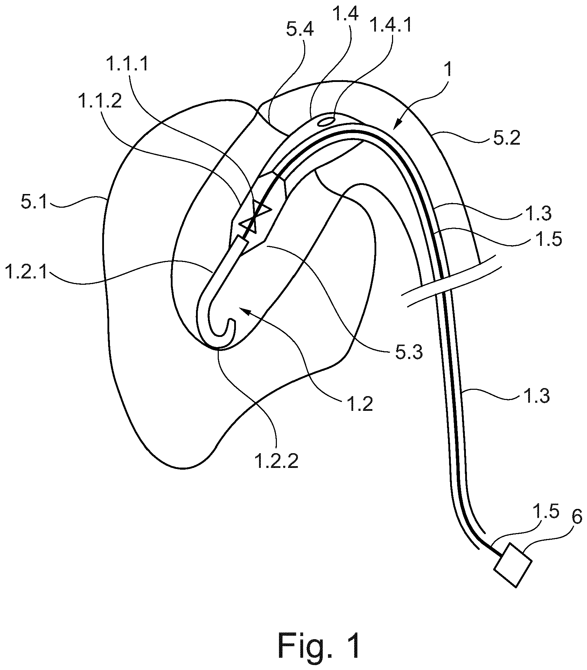

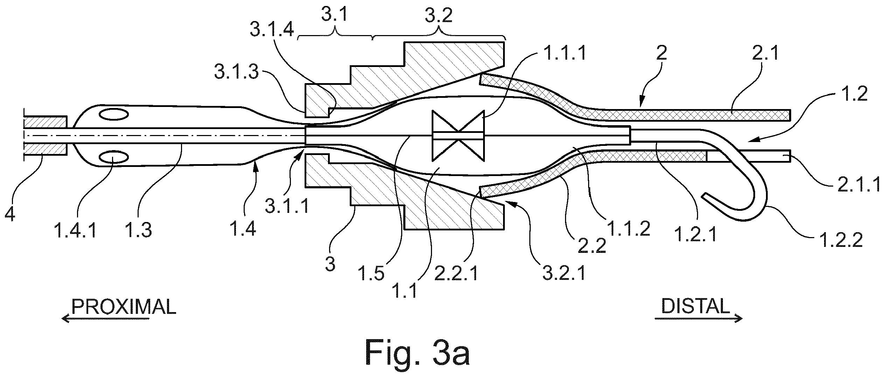

Figure 1 shows a catheter device 1 used as a blood pump. The catheter device 1 is introduced into a patient, such that a portion of the distal end region of the catheter device 1 is positioned within the left ventricle 5.3 of the heart 5.1 of the patient. In a driving region, which can lie outside of the patient's body, a motor 6 is provided for driving a drive shaft 1.5. A portion of the drive shaft 1.5 is covered by the catheter tube 1.3. The drive shaft 1.5 and the catheter tube 1.3 extend from the driving region to the distal end region, where a radially compressible rotor 1.1.1 is driven by the drive shaft 1.5. The compressible rotor 1.1.1 is located within a compressible housing 1.1.2. The compressibility of the rotor 1.1.1 and the housing 1.1.2 is useful for introducing the rotor into the patient's body. During operation, the rotor 1.1.1 and the housing 1.1.2 are in an expanded state. The housing 1.1.2 prevents damage to heart tissue such as for instance the tendinous chords, as it prevents tissue from being sucked into the rotor 1.1.1 or becoming entangled with the rotor 1.1.1 or the drive shaft 1.5. The distal end of the drive shaft 1.5 lies within a distal bearing 1.2. The catheter device 1 can further comprise an atraumatic tip, for instance designed as a pigtail tip 1.2.2. The atraumatic tip 1.2.2 can for instance be made of Pebax ^{®}, PU, or another flexible medical grade polymer and it can comprise an elongated portion 1.2.1. In the embodiment shown in Figure 3a, the distal end of the drive shaft is borne in the elongated portion 1.2.1. The distal bearing 1.2 and the atraumatic tip can however also be provided separately. The rotor 1.1.1 and the drive shaft 1.5 can rotate such that a flow of blood away from the distal end, towards the proximal end is effected, i.e. a blood flow out of the left ventricle 5.3 into the aorta 5.2 and to other regions of the patient's body. A downstream tubing 1.4 is provided proximally of the rotor 1.1.1 and the rotor housing 1.1.2. The downstream tubing 1.4 has a downstream opening 1.4.1 that lies proximally of the aortic valve 5.4, such that the blood passes the aortic valve within the downstream tubing 1.4 and can then stream into the aorta 5.2. The downstream tubing 1.4 is made of a flexible material, such that it can be compressed by the aortic valve 5.4 as the patient's heart 5.1 continues to pump.

Figure 2 shows the distal end region of a catheter device with a radially compressible part 1.1. The radially compressible part 1.1 comprises a radially compressible rotor 1.1.1 and a radially compressible housing 1.1.2. The rotor 1.1.1 can for instance be inserted into the left ventricle of a human heart and is designed to be driven by means of a drive shaft 1.5 to effect a flow of fluid in a proximal direction, away from the distal bearing, into a downstream tubing 1.4 provided proximally of the rotor, and out of the downstream opening 1.4.1 of the downstream tubing 1.4, for instance into the aorta of a human. Distally of the compressible part 1.1, a distal bearing 1.2 is provided. The bearing comprises an elongated portion 1.2.1 for rotatably mounting the drive shaft 1.5 and a flexible pig tail 1.2.2.

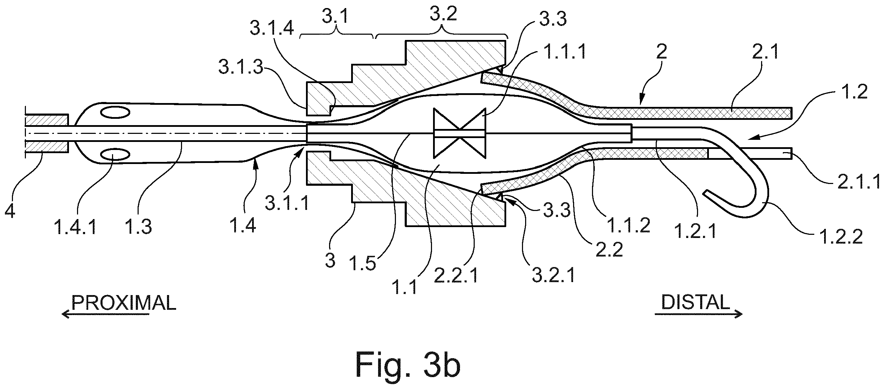

Figure 3 a shows the distal end region of the catheter device as shown in Figure 1, with an additional compression pipe 2 and a reducer 3 to aid the insertion of the catheter device, in particular the compressible part 1.1, into a sleeve 4. The sleeve 4 is provided around the catheter device 1, proximally of the compressible part 1.1. The compression pipe 2 is provided around a portion of the compressible part 1.1, wherein the movable fins 2.2 lie around a distal portion of the compressible part 1.1 and the tube 2.1 of the compression pipe 2 extends distally thereof. The pipe 2 surrounds the elongated portion 1.2.1 of the distal bearing 1.2, wherein a recess 2.1.1 of the compression pipe 2 allows the pigtail 1.2.2 of the distal bearing 1.2 to remain in the curled position. The Reducer 3 is provided proximally of the compression pipe. The proximal portion of the reducer 3 is a cylindrical portion 3.1 and the distal portion of the reducer 3 is a conical portion 3.2, the radius of which increases in distal direction. The aperture angle of the conical portion 3.2 can for instance be between 6° and 10°. The reducer can now be moved in the distal direction, with respect to the catheter device 1 and the compression pipe 2. The distal opening 3.2.1 of the reducer 3 is large enough for the movable fins 2.2 to slide into the distal opening 3.2.1 in the open state. Frictional forces and/or the pigtail 1.2.2 residing in the recess 2.1.1 ensure that the compression pipe 2 does not slide of the catheter device 1 on the distal end. As the relative movement continues, and the movable fins 2.2 slide through the conical portion 3.2 of the reducer 3, the movable fins enter in contact with the conical inner wall 3.2.2 of the conical portion 3.2 of the reducer 3 and are therefore continuously compressed. As the movable fins 2.2 are compressed, the radially compressible part 1.1 which is provided between the movable fins 2.2 is also compressed and the compression force acts preferably in an axial section, where the rotor is located. As the proximal end of the compression pipe 2 enters the cylindrical portion 3.1, compression is completed. The compression pipe 2 slides further, until a front part 2.2.1 of the movable fins strikes a second stop 3.1.4 near the proximal opening 3.1.1 of the reducer 3, opposing the first stop 3.1.3. The compression pipe 2 can now not be moved further in the proximal direction on the catheter device 1. The sleeve 4 can now be provided against the first stop 3.1.3 or against the front part 2.2.1 of the movable fins 2.2. By holding on to the sleeve 4 and pulling on a proximal end of the catheter tube, the compressible part 1.1 is pulled out of the compression pipe 2, through the proximal opening 3.1.1 of the reducer 3, and into the sleeve 4.

Figure 3 b shows the same setup as Fig. 3 a, but with an additional set of stops 3.3 for holding the compression pipe 2 in an uncompressed state in place with respect to the reducer 3. The catheter device 1 can be packaged and delivered to the practitioner in this configuration, with the compressible rotor 1.1.1 and housing 1.1.2 in the uncompressed state, but the compression pipe 2 and the reducer 3 in a configuration as shown in Figure 3 b, such that the compressible part is protected.

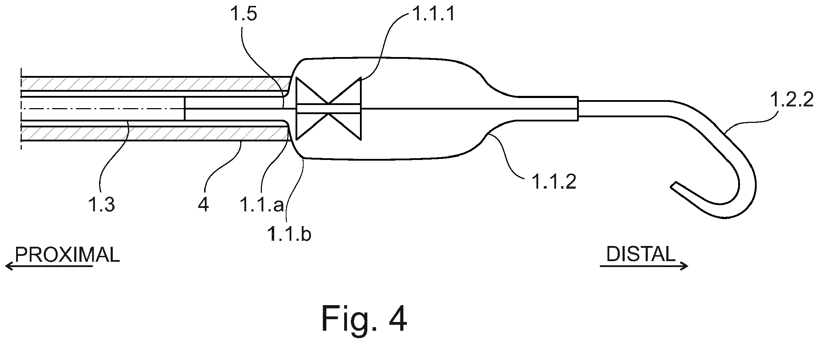

Figure 4 shows the distal end region of the catheter device 1 being pulled into a sleeve 4 according to the prior art. The housing 1.2 and the rotor 1.1 are thereby compressed as they are squeezed into the sleeve 4. The compression force is mediated directly by the edges of the sleeve, resulting in strong deformation of the compressible part, at least in the regions 1.1.a and 1.1.b illustrated in Figure 3. Furthermore, the force acting on the compressible part 1.1 acts in the distal direction, the rotor 1.1.1 is therefore pushed towards the distal end and cannot expand axially in the proximal direction as it is compressed. This can lead to inaccurate folding of the rotor or kinks in the compressed rotor.

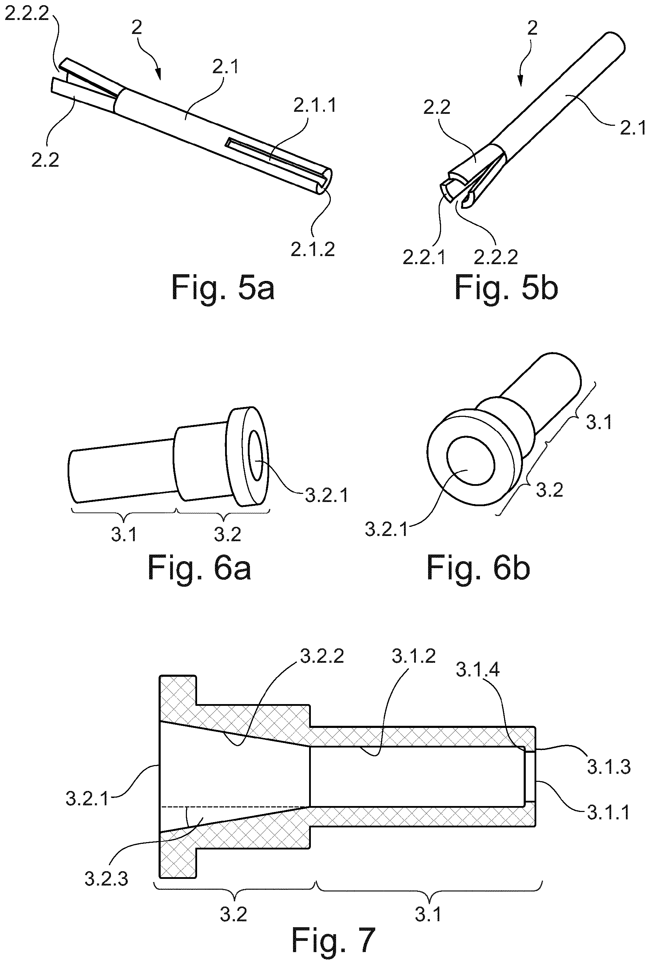

Figures 5a and 5b show the compression pipe 2 from two different perspectives. The compression pipe 2 comprises three movable fins 2.2 which are shown in an open position. In the open position, a gap 2.2.2 is found between neighboring fins. The movable fins 2.2 are preferably designed such that upon compression, in particular compression by means of a reducer 3 as shown in Figure 3, said gap 2.2.2 does not fully close. By having a residual gap 2.2.2 when the fins are in the closed state, a compressible part 1.1 or other parts of the catheter device such as the downstream tubing 1.4 cannot be pinched or caught between the fins. In Figure 5a, the recess 2.1.1 is visible, which allows for a pigtail 1.2.2 to remain in the curled position when the compression pipe 2 is provided around a distal bearing 1.2 or a pigtail 1.2.2. Thus, different sizes of catheter devices, with different lengths of distal bearings, can be accommodated. Also, providing the pigtail 1.2.2 in a recess, rather than the pigtail protruding past the distal end of the compression pipe, protects the pigtail 1.2.2 at least in part from being bent laterally. In Figure 5b, the front part 2.2.1 of the movable fins 2.2 is visible, which is designed to strike a second stop 3.1.4 of the reducer 3.

Figures 6a and 6b show the reducer 3 from two different perspectives. The reducer 3 comprises a cylindrical portion 3.1, and a conical portion 3.2. The reducer is typically provided around a catheter device 1, such that the cylindrical portion lies proximally of the conical portion. The inner radius of the conical portion 3.2 increases away from the cylindrical portion 3.1, towards a distal opening 3.2.1.

In Figure 7, a cut through the reducer 3 is shown. The distal opening 3.2.1, of the conical portion 3.2 of the reducer 3 is shown on the left. The inner radius of the conical portion 3.2 decreases away from the distal opening 3.2.1, such that the inner wall 3.2.2 describes a section of a circular conical surface. To the right of the conical portion 3.2, the cylindrical portion is shown. The inner wall 3.1.2 of the cylindrical portion describes a cylindrical surface. The opening angle 3.2.3 of the wall 3.2.2 of the conical portion 3.2 with respect to the wall 3.1.2 of the cylindrical portion 3.1 can for instance be, between 6° and 10°. The cylindrical section 3.1 has a proximal opening 3.1.1. Near the proximal opening, a first stop 3.1.3 on the proximal side and an opposing second stop 3.1.4, on the distal side, are provided. The reducer 3 is designed such that the movable fins 2.2 can be introduced into the distal opening 3.2.1 in an uncompressed or almost uncompressed state. If the compression pipe 2 slides along the reducer 3 in proximal direction, the movable fins 2.2 are radially isotropically compressed as the fins 2.2 slide through the conical portion 3.2 of the reducer 3. Once the movable fins 2.2 reach the cylindrical portion 3.1, compression is completed. The fins 2.2 can now slide further until they strike the second stop 3.1.4. The diameter of a cylindrical space contained within the movable fins 2.2 in the compressed state differs preferably by less than 1mm from the inner diameter of the proximal opening 3.1.1, particularly preferably by less than 0.5 mm, such that a compressible part 1.1 can slide out of said cylindrical space and through the proximal opening 3.1.1 and into the sleeve 4. The sleeve 4 can thereby be provided proximally of the reducer 3, against the first stop 3.1.3.

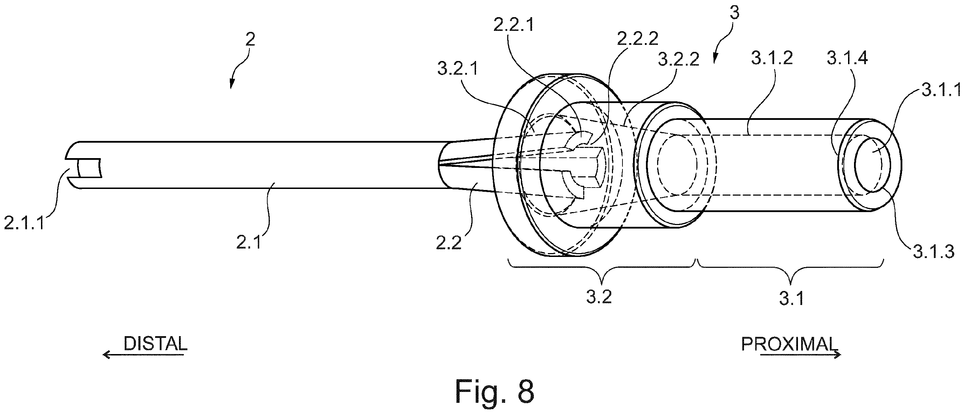

Figure 8 shows the compression pipe 2 and the reducer 3 without the catheter device 1. The movable fins 2.2 have been inserted through the distal opening 3.2.1 of the reducer, into the conical portion 3.2 of the reducer 3, i.e., the fins 2.2 are slightly compressed due to the conical inner wall 3.2.2. The compression pipe is oriented such that the tube 2.1 is located distally of the movable fins 2.2. The recess 2.1.1 can be seen near the distal end of the tube 2.1. On the proximal side of the setup, the cylindrical portion 3.1 of the reducer can be seen, with the proximal opening 3.1.1 and the first stop 3.1.3 and the second stop 3.1.4.

Figures 9 a and 9 b show the catheter device 1, the compression pipe 2 and the reducer 3 and an additional disk device 3.4 or membrane with a hole.

In Figure 9 a, the disk device 3.4 is provided around the drive shaft 1.5 and the catheter tube 1.3 between the reducer 3 and the compressible part 1.1. As the reducer 3 is moved towards the compression pipe 2 and the compressible part, the front part of reducer 3 near the distal opening 3.2.1 of the reducer, enters in contact with the disk device 3.4, and thereby pushes the disk device 3.4 towards the compression pipe 2 and the compressible part 1.1. As the disk device 3.4 thus approaches the compressible part 1.1, the disk device 3.4 pushes the compressible part 1.1 between the movable fins 2.2 of the compression pipe, thus positioning the compressible part such that the compression force during the subsequent compression can act at the desired position. The movable fins 2.2 and the distal opening 3.2.1 of the reducer 3 are designed such that the movable fins 2.2 enter smoothly into the distal opening 3.2.1 of the reducer when the uncompressed radially compressible part 1.1 is at said desired position. As the relative movement of the reducer 3 and the disk device 3.4 with respect to the compression pipe 2 continues, the disk device 3.4 gives in or breaks, such that the compression pipe 2 with the radially compressible part 1.1 received therein inter the conical portion of the reducer.

Figure 9 b shows a similar setup as Figure 9 a, but with the disk device 3.4 attached to the reducer 3. The disk device 3.4 is provided in the distal opening 3.2.1 of the reducer. The handling is similar to the handling described in the context of Figure 9 a. As the reducer 3 and the disk device 3.4 approach the compression pipe 2 and the radially compressible part 1.1, the radially compressible part is first pushed between the movable fins 2.2 and positioned at a desired location, wherein the movable fins 2.2 are starting to slide into the conical portion of the reducer 3, but are not yet being compressed or only slightly compressed. As the relative movement of the reducer 3 with respect to the compression pipe 2 continues, the disk gives in or breaks and the movable fins 2.2 and thus the radially compressible part 1.1 are compressed as they slide through the conical portion of the reducer 3.

Figure 9 c shows the disk device 3.4 from Figures 9 a and 9 b. The disk device 3.4 is preferably configured as a thin plate. It can for example be made of PTFE, PE; or Silicone. The disk device 3.4 comprises a hole 3.4.1 at the center or near the center. The disk device 3.4 can comprise a slit which runs from the hole 3.4.1 to the outer edge of the disk device 3.4, such that the disk device can be clipped or mounted around the catheter tube 1.3. The disk device 3.4 comprises no sharp edges at the rim of the hole 3.4.1 or along the slit 3.4.2, such that the catheter device 1 or the catheter tube 1.3 is not damaged when the disk device 3.4 is clipped on. The disk device, in particular the edge of the hole 3.4.1 are designed such that the disk exhibits good sliding properties on the catheter. The disk device 3.4 can for example be circular. The radius or radial extension of the disk device is larger than the radius of the cylindrical portion of the reducer. Preferably, the radius or radial extension of the disk device is larger than the radius of a circular plane circumscribed by the front parts of the movable fins in the uncompressed state. The disk device 3.4 is flexible such that it can give in and/or fall off as the disk device is pushed into the conical portion of the reducer 3.

Figure 10 shows the distal end region of the catheter device with the compression pipe 2 and the reducer 3, similarly to Figure 3. However, in Figure 10, the compression pipe is provided in an alternative configuration. The compression pipe 2 is reversed with respect to the configuration from Figure 3. The movable fins 2.2 of the compression pipe are now provided around the compressible part 1.1 from the proximal side, such that the tube 2.1 surrounds a portion of the catheter device 1 that lies proximally of the compressible part 1.1 and the distal bearing 1.2 is exposed. The reducer 3 is provided around the compression pipe 2 with the conical portion 3.2 distally of the cylindrical portion 3.1. The catheter device 1 and the compression pipe 2 are now moved in a proximal direction, relative to the reducer 3, such that the tube 2.1 enters the reducer first. When the movable fins 2.2 with the compressible part enter the conical portion 3.2 of the reducer, the compression of the compressible part 1.1 starts. In this configuration, the compressible part 1.1 is pulled into the compression pipe 2 when a pulling force is exerted on the proximal end of the catheter tube 1.3. Thus, different from the configuration from Figure 3, no frictional forces are needed to preserve the relative position of the catheter device 1 and the compression pipe 2. The reducer 3 can be designed without stops at the proximal opening. The sleeve 4 can then be provided directly on the proximal end of the compression pipe 2. A configuration with stops, as shown in Figures 2, 6 and 7, is also possible. Then, the cylindrical portion 3.1 of the reducer should be long enough to entirely accommodate the compression pipe 2, i.e., both the tube 2.1 and the movable fins 2.2, such that the movable fins 2.2 can fully enter the cylindrical portion 3.1 of the reducer 3 and be fully compressed.

Figure 11 a and 11 b shows the rotor 1.1.1 and the housing 1.1.2 and the sleeve 4 in two states, uncompressed (a) and compressed (b). When the rotor 1.1.1 and the housing 1.1.2 are transferred into the sleeve 4, the rotor 1.1.1 and the housing 1.1.2 are compressed in a radial direction, from their expanded states 1.1.1, 1.1.2 into their compressed states 1.1.1', 1.1.2'. The rotor 1.1.1 in the expanded state has an axial length 1.1.3. As the rotor 1.1.1 is compressed to the compressed state 1.1.1', the axial length increases to a length 1.1.3'. Similarly, the housing 1.1.2 in the expanded state has an axial length 1.1.4. As the housing 1.1.2 is compressed to the compressed state 1.1.2', the axial length increases to a length 1.1.4'. The elongation of the rotor allows for a better compression than if the length is kept constant. If the compression force has a larger radial component, as in the case when the compression pipe 2 and the reducer 3 are used, the rotor 1.1.1 can expand axially. On the other hand, if the rotor is simply pulled into a sleeve 4 as shown in Figure 3, axial elongation of the rotor 1.1.1 is hindered which can result in faulty folding of the rotor and/or kinks in the rotor.

Figure 12 shows the reducer 3, a part of the compression pipe 2, a part of the sleeve 4 and the radially compressible part 1.1 with the rotor 1.1.1' and the housing 1.1.2' in the compressed state. The rotor 1.1.1' and the housing 1.1.2' are provided between the movable fins 2.2 of the compression pipe, the movable fins 2.2 being fully introduced into the cylindrical portion 3.1 of the reducer and, thus, in the fully compressed position. A front part 2.2.1 of the movable fins 2.2 abuts a first stop 3.1.4 of the reducer, such that the compression pipe 2 cannot slide all the way through the reducer 3. In the embodiment depicted in Figure 12, the front part 2.2.1 of the movable fins 2.2 acts as a stop for the sleeve 4 provided around the drive shaft 1.5 and the catheter tube 1.3. By holding on to the sleeve 4 and pulling on the catheter tube 1.3 in the proximal direction, the compressible part slides out of the compression tube 2 and the reducer 3, into the sleeve 4.

Figure 13 shows a compressible housing 1.1.2 which features a lower density of struts 1.1.5 near the proximal end of the housing 1.1.2 and a higher density of struts 1.1.5' near the distal end of the housing 1.1.5. In a preferred mode of operation, a flow of blood in proximal direction is effected. Due to the low density of struts in the proximal end region, collision of blood cells with the struts is minimized and thus damage to the blood is minimized. However, in such a configuration, if the compressible housing 1.1.2, with a rotor 1.1.1 on the inside, is pulled into the sleeve 4 provided proximally of the housing 1.1.2, the risk of the rotor 1.1.1 being pinched or caught between the struts increases, if the compression is mediated only by the end of the sleeve 4. Therefore, a setup with a reducer 3 and a compression pipe 2 as described in the application is particularly useful, if a housing design as shown in Fig. 13 is desired.

Figure 14a shows the sleeve 4 and the compression pipe 2 that are introduced into the reducer 3. The reducer exposes a step inside the cylindrical portion thereof, such that an inner diameter is reduced at the cylindrical end, into which end the sleeve is introduced. The inner diameter is thus slightly larger at the end of the cylindrical portion facing the conical portion, i.e., in the part of the cylindrical portion into which the compression pipe is introduced. In the embodiment shown in Figure 14, the compression pipe 2 has an additional stop 2.3, abutting against the end of the reducer 3 on the side of the conical portion. The stop 2.3 is configured such that a gap 2.5 remains between the compression pipe and the step provided on the inside of the reducer 3, near the end of the movable fins 2.2 of the compression pipe. This way, parts of the catheter device 1 cannot be pinched between the front side 2.2.1 of the movable fins 2.2 and the step when the catheter device is pulled through the compression pipe 2. The sleeve 4 also has an additional stop 4.1 which abuts against the end of the cylindrical portion of the reducer 3, such that the end of the sleeve 4 that is introduced into the reducer 3 remains at a distance from the front side 2.2.1 of the movable 2.2 fins. This way, the gap 2.5 persists also when the sleeve 4 is introduced into the reducer 3.

The inner diameter of the part of the cylindrical portion of the reducer into which the sleeve 4 is introduced is between 0.2 and 1 mm, especially between 0.4 mm and 0.7 mm, larger than an outer diameter of the sleeve 4. This way, deforming of the sleeve 4 is tolerated when the catheter device is pulled into the sleeve 4. This is particularly advantageous as the rotor 1.1.1 is sometimes compressed to obtain an ellipsoid shape, rather than a circular shape.

The compression pipe 2 does not comprise a recess for receiving the pigtail 1.2.2 of the catheter device 1.

The step provided on the inside of the reducer 3 of Fig. 14a can also have a rounded shape or it can be designed as a slope. Fig. 14b shows such an arrangement with a transition zone 2.6 which does not have sharp edges. By pulling the pump head through this transition zone 2.6, a further reduction of the diameter of the pump head can be achieved.

Figure 15 shows an embodiment of the compression pipe 2, wherein additional slits 2.4 are provided, perforating the compression pipe 2 in several places. The compression pipe 2 has four movable fins 2.2 separated by gaps 2.2.2. Slits 2.4 are provided both as a continuation of the gaps 2.2. -- i.e., radially aligned with the gaps -- and shifted with respect to the gaps 2.2.2 -- i.e., radially displaced to lie between two gaps 2.2.2. This way, the flexibility of the compression pipe is improved, enabling better compression behavior and helping to avoid pinching of parts of the catheter device 1.

The compression pipe 2 shown in Figure 15 also exhibits the additional stop 2.3 as shown in and discussed in the context of Fig. 14.

Figures 16 a-e show different embodiments of the compression pipe 2, differing from each other in how the gaps 2.2.2 and slits 2.4 are arranged. In each case, the additional stop 2.3 is provided. The compression pipe 2 is shown in a compressed state.

In each case, the compression pipe 2 has four movable fins 2.2. The width of the fins 2.2 is chosen to be large enough to engage with the struts of the compressible housing of the catheter device 1. I.e., for a higher density of struts, thinner fins can be provided. When the density of struts is low in all parts of the housing, broader fins are provided, sometimes embodiments with three fins instead of four fins are chosen for a low density of struts.

Fig 16a shows an embodiment in which the gaps 2.2.2 separating the fins 2.2 extend axially along the compression pipe.

Fig. 16b shows an embodiment in which the gaps 2.2.2 start in the longitudinal direction and then extend with a radial component, i.e. run in a spiral fashion. The gaps run about 45° around the circumference of compression pipe. The risk of pinching of the catheter device can be reduced by having a radial component for the gaps 2.2.2.

Fig. 16c also shows gaps 2.2.2 with a radial component. The gaps run around the circumference of the compression pipe, covering about 90°.

Fig. 16d shows an embodiment with axially extending gaps 2.2.2 and having additional slits 2.4. The embodiment is similar to the one shown in Fig. 15. Slits 2.4 are provided both radially aligned with the gaps 2.2.2, distanced from the gaps, and in between neighboring gaps, distanced from the end of the compression pipe.

Fig. 16e shows an embodiment having both gaps 2.2.2 with a radial component and additional slits 2.4. The slits 2.2.2 show the same spiral course as the gaps 2.2.2, some slits being aligned with the spiral course of one of the gaps, other slits being arranged in between the spirals defined by the gaps 2.2.2. Ends of radially displaced slits 2.7 overlap axially with ends of gaps 2.2.2 and/or axially aligned slits 2.4 (this is also shown in Figs. 15 and 16).

The invention is defined by the claims. The following aspects form part of the present disclosure and are helpful for understanding the invention:
1. System for introducing a radially compressible part (1.1) of a catheter device (1) into a sleeve (4), comprising
   a sleeve (4),
   a catheter device (1) comprising a radially compressible part (1.1), the radially compressible part (1.1) being configured to be transferred at least in part into the sleeve (4);
   a compression pipe (2) which comprises movable fins (2.2) for receiving the radially compressible part (1.1), the movable fins (2.2) having an open state and a closed state.
2. System according to aspect 1, wherein the radially compressible part (1.1) comprises a radially compressible rotor (1.1.1) and a radially compressible housing (1.1.2).
3. System according to one of the preceding aspects, wherein the sleeve (4) is a peel-away sheath or the sleeve (4) is a cannula.
4. System according to aspect 3, wherein the cannula pertains to the catheter device (1) itself.
5. System according to one of the preceding aspects, wherein the catheter device (1) comprises a pigtail (1.2.2) and/or a distal bearing (1.2), the distal bearing or the pigtail (1.2.2) comprising an elongated portion (1.2.1).
6. System according to one of the preceding aspects, wherein the compression pipe (2) comprises a tube (2.1) for receiving the elongated portion (1.2.1) or a portion of the catheter device lying proximally or distally of the radially compressible part (1.1).
7. System according to one of the preceding aspects, wherein the tube (2.1) of the compression pipe (2) comprises a recess (2.1.1) for receiving the pigtail (1.2.2).
8. System according to one of the preceding aspects, wherein the compression pipe (2) comprises between two and ten movable fins (2.2).
9. System according to one of the preceding aspects, wherein the compression pipe (2) comprises three or four movable fins (2.2).
10. System according to one of the preceding aspects, wherein gaps (2.2.2) extending between neighboring movable fins (2.2) extend axially or spirally.
11. System according to aspect 10, wherein slits (2.4) are provided in the compression pipe (2), the slits (2.4) extending axially or spirally, as continuations of the gaps (2.2.2) or in between the gaps (2.2.2).
12. System according to aspect 11, wherein ends of radially displaced slits (2.7) overlap axially with ends of gaps (2.2.2) and/or axially designed slits (2.4)
13. System according to one of the preceding aspects, wherein a gap remains between neighboring movable fins (2.2) in the closed state.
14. System according to one of the preceding aspects, wherein neighboring fins (2.2) are connected by a membrane or skin.
15. System according to one of the preceding aspects, further comprising a reducer (3) for radially compressing the movable fins (2.2) of the compression pipe (2) and the radially compressible part (1.1) received between the movable fins (2.2).
16. System according to aspect 15, wherein the reducer (3) is designed as a pipe with a distal (3.2.1) and a proximal opening (3.1.1) and configured to receive the sleeve (4) and the compression pipe (2), and wherein the sleeve (4) comprises a stop (4.1) configured to abut against an end of the reducer (3) when the sleeve (4) is introduced into the proximal opening (3.1.1) of the reducer (3) and/or wherein the compression pipe (2) comprises a stop (2.3) configured to abut against an end of the reducer when the compression pipe (2) is introduced into the distal opening (3.1.2) of the reducer (3), such that a gap (2.5) remains between the sleeve (4) and the compression pipe (2) when the compression pipe (2) and the sleeve (4) are introduced into the reducer.
17. System according to one of the preceding aspects, wherein the reducer (3) is designed as a pipe with a distal (3.2.1) and a proximal opening (3.1.1), comprising a proximal cylindrical portion (3.1) of constant inner diameter and a distal conical portion (3.2), where the inner diameter increases in distal direction, the distal conical portion (3.2) configured for receiving the movable fins (2.2) and the radially compressible part (1.1) received by the movable fins (2.2).
18. System according to one of the preceding aspects, wherein the reducer (3) comprises a first stop (3.1.3) for limiting the movement of the sleeve (4) with respect to the reducer (3) in the distal direction.
19. System according to one of the preceding aspects, wherein the reducer (3) comprises a second stop (3.1.4) opposite to the first stop (3.1.3) for limiting the movement of the compression pipe (2) with respect to the reducer (3) in the proximal direction.
20. System according to one of the preceding aspects, wherein an inner diameter of the movable fins (2.2) in the closed state and an inner diameter of the sleeve (4) differ by at most 1 mm, preferably by at most 0.5 mm.
21. System according to one of aspects 15 to 20, wherein the compression pipe (2) is provided around the catheter device (1), such that the movable fins (2.2) face the compressible part (1.1) and the reducer (3) is provided on an opposite side of the compressible part (1.1), such that the conical portion (3.2) of the reducer (3) faces the compressible part (1.1), further comprising a disk device (3.4) with a hole (3.4.1), characterized in that the disc device (3.4) is provided around the catheter device (1) in a section between the cylindrical portion (3.1) of the reducer (3) and the compressible part (1.1).
22. Method for using a system of one of aspects 15 to 21 for inserting the radially compressible part (1.1) of the catheter device (1) into the sleeve (4), the method comprising the steps of:
   providing the reducer (3) around the catheter device (1) proximally of the compressible part (1.1);
   providing the sleeve (4) around the catheter device (1) proximally of the reducer (3);
   providing the compression pipe (2) around the catheter device (1), such that the radially compressible part (1.1) is located between the movable fins (2.2);
   and executing the following steps:
      moving the reducer (3) towards the compression pipe (2), such that the compression pipe (2) slides into the distal opening of the reducer (3);
      continuing the relative movement of the compression pipe (2) and the reducer (3) such that the movable fins (2.2) slide through the distal conical portion (3.2) of the reducer (3), effecting compression of the movable fins (2.2) from the open state to the closed state, thus compressing the radially compressible part (1.1);
      continuing the relative movement such that the movable fins (2.2) in the compressed state and the compressed radially compressible part (1.1) slide along the proximal cylindrical portion (3.1); and
      providing the sleeve (4) proximally against the compression pipe (2) or against a stop (3.14) pertaining to the reducer (3), the stop being provided proximally of the compression pipe (2), and pulling the catheter tube (1.3) in a proximal direction while holding on to the sleeve (4), such that at least the portion of the catheter device (1) comprising the radially compressible part (1.1) slides out of the compression pipe (2) and into the sleeve (4).
23. Method for using the system of aspect 21 for inserting the radially compressible part (1.1) of the catheter device (1) into the sleeve (4), the method comprising the steps of:
   providing the reducer (3) around the catheter device (1) proximally of the compressible part (1.1);
   providing the sleeve (4) around the catheter device (1) proximally of the reducer (3);
   providing the compression pipe (2) around the catheter device (1), such that the movable fins (2.2) face in the proximal direction;
   and executing the following steps:
      moving the the reducer (3) and the disk device (3.4) towards the compression pipe (2), such that the disk device (3.4) pushes the compressible part (1.1) between the movable fins (2.2) and sliding the movable fins (2.2) into the distal opening of the reducer (3) until the disk device (3.4) gives in or breaks;
      continuing the relative movement of the compression pipe (2) and the reducer (3) such that the movable fins (2.2) slide through the distal conical portion (3.2) of the reducer (3), effecting compression of the movable fins (2.2) from the open state to the closed state, thus compressing the radially compressible part (1.1);
      continuing the relative movement such that the movable fins (2.2) in the compressed state and the compressed radially compressible part (1.1) slide along the proximal cylindrical portion (3.1); and
      providing the sleeve (4) proximally against the compression pipe (2) or against a stop (3.14) pertaining the reducer (3), the stop being provided proximally of the compression pipe (2), and pulling the catheter tube (1.3) in a proximal direction while holding on to the sleeve (4), such that at least the portion of the catheter device (1) comprising the radially compressible part (1.1) slides out of the compression pipe (2) and into the sleeve (4).

### List of reference numerals

- 1: Catheter device
- 1.1: Radially compressible part
- 1.1.a: Region of strong deformation
- 1.1.b: Region of strong deformation
- 1.1.1: Radially compressible rotor
- 1.1.1': Radially compressible rotor (compressed state)
- 1.1.2: Radially compressible housing
- 1.1.2': Radially compressible housing (compressed state)
- 1.1.3: Length of radially compressible rotor
- 1.1.3': Length of radially compressible rotor (compressed state)
- 1.1.4: Length of radially compressible housing
- 1.1.4': Length of radially compressible housing (compressed state)
- 1.1.5: Struts of compressible housing (low density)
- 1.1.5': Struts of compressible housing (high density)
- 1.2: Distal bearing
- 1.2.1: Elongated portion of the distal bearing
- 1.2.2: Pigtail
- 1.3: Catheter tube
- 1.4: Downstream tubing
- 1.4.1: Downstream opening
- 1.5: Drive shaft
- 2: Compression pipe
- 2.1: Tube
- 2.1.1: Recess
- 2.2: Movable fins
- 2.2.1: Front part of the movable fins
- 2.2.2: Gap between neighboring movable fins
- 2.3: Stop
- 2.4: Slit
- 2.5: Gap
- 2.6: Transition zone
- 2.7: Slit
- 3: Reducer
- 3.1: Proximal cylindrical portion
- 3.1.1: Proximal opening
- 3.1.2: Cylindrical inner wall
- 3.1.3: First stop
- 3.1.4: Second stop
- 3.2: Distal conical portion
- 3.2.1: Distal opening
- 3.2.2: Conical inner wall
- 3.2.3: Opening angle of the conical inner wall
- 3.3: Third stop
- 3.4: Disk device
- 3.4.1: Hole
- 3.4.2: Slit

- 4: Sleeve
- 4.1: Stop

- 5.1: Heart
- 5.2: Aorta
- 5.3: Left ventricle
- 5.4: Aortic valve

- 6: Motor

## Claims

1. System for introducing a radially compressible part (1.1) of a catheter device (1) into a sleeve (4), comprising
a sleeve (4),
a catheter device (1) comprising a radially compressible part (1.1), the radially compressible part (1.1) being configured to be transferred at least in part into the sleeve (4);
a compression pipe (2) which comprises movable fins (2.2) for receiving the radially compressible part (1.1), the movable fins (2.2) having an open state and a closed state.

2. System according to claim 1, wherein the radially compressible part (1.1) comprises a radially compressible rotor (1.1.1) and a radially compressible housing (1.1.2).

3. System according to claim 2, wherein an opening angle of the movable fins (2.2) in an open state is such that the movable fins (2.2) can be brought in contact with an axial section of the radially compressible housing (1.1.2) of the radially compressible part (1.1), which axial section lies between the distal end and the proximal end of the radially compressible housing (1.1.2), such that when the movable fins (2.2) are transferred to the closed state, the radially compressible part (1.1) is compressed and a compression force is applied at said axial section, wherein the compression force has a radial component.

4. System according to one of the preceding claims, wherein the sleeve (4) is a peel-away sheath or the sleeve (4) is a cannula, the cannula (4) optionally pertaining to the catheter device (1) itself.

5. System according to one of the preceding claims, wherein the catheter device (1) comprises a pigtail (1.2.2) and/or a distal bearing (1.2), the distal bearing or the pigtail (1.2.2) comprising an elongated portion (1.2.1).

6. System according to one of the preceding claims, wherein the compression pipe (2) comprises a tube (2.1) for receiving the elongated portion (1.2.1) or a portion of the catheter device lying proximally or distally of the radially compressible part (1.1), wherein the tube (2.1) of the compression pipe (2) optionally comprises a recess (2.1.1) for receiving the pigtail (1.2.2).

7. System according to one of the preceding claims, wherein the compression pipe (2) comprises between two and ten movable fins (2.2), in particular three or four movable fins (2.2).

8. System according to one of the preceding claims, wherein gaps (2.2.2) extending between neighboring movable fins (2.2) extend axially or spirally.

9. System according to claim 8, wherein slits (2.4) are provided in the compression pipe (2), the slits (2.4) extending axially or spirally, as continuations of the gaps (2.2.2) or in between the gaps (2.2.2), wherein, preferably, ends of radially displaced slits (2.7) overlap axially with ends of gaps (2.2.2) and/or axially designed slits (2.4).

10. System according to one of the preceding claims, wherein a gap remains between neighboring movable fins (2.2) in the closed state and/or wherein neighboring fins (2.2) are connected by a membrane or skin.

11. System according to one of the preceding claims, further comprising a reducer (3) for radially compressing the movable fins (2.2) of the compression pipe (2) and the radially compressible part (1.1) received between the movable fins (2.2).

12. System according to claim 11, wherein the reducer (3) is designed as a pipe with a distal (3.2.1) and a proximal opening (3.1.1) and configured to receive the sleeve (4) and the compression pipe (2), and wherein the sleeve (4) comprises a stop (4.1) configured to abut against an end of the reducer (3) when the sleeve (4) is introduced into the proximal opening (3.1.1) of the reducer (3) and/or wherein the compression pipe (2) comprises a stop (2.3) configured to abut against an end of the reducer when the compression pipe (2) is introduced into the distal opening (3.1.2) of the reducer (3), such that a gap (2.5) remains between the sleeve (4) and the compression pipe (2) when the compression pipe (2) and the sleeve (4) are introduced into the reducer and/or
wherein the reducer (3) is designed as a pipe with a distal (3.2.1) and a proximal opening (3.1.1), comprising a proximal cylindrical portion (3.1) of constant inner diameter and a distal conical portion (3.2), where the inner diameter increases in distal direction, the distal conical portion (3.2) configured for receiving the movable fins (2.2) and the radially compressible part (1.1) received by the movable fins (2.2) and/or
wherein the reducer (3) comprises a first stop (3.1.3) for limiting the movement of the sleeve (4) with respect to the reducer (3) in the distal direction
and/or
wherein the reducer (3) comprises a second stop (3.1.4) opposite to the first stop (3.1.3) for limiting the movement of the compression pipe (2) with respect to the reducer (3) in the proximal direction.

13. System according to one of claims 11 or 12, wherein the compression pipe (2) is provided around the catheter device (1), such that the movable fins (2.2) face the compressible part (1.1) and the reducer (3) is provided on an opposite side of the compressible part (1.1), such that the conical portion (3.2) of the reducer (3) faces the compressible part (1.1), further comprising a disk device (3.4) with a hole (3.4.1), **characterized in that** the disc device (3.4) is provided around the catheter device (1) in a section between the cylindrical portion (3.1) of the reducer (3) and the compressible part (1.1).

14. Method for using a system of one of claims 11 to 13 for inserting the radially compressible part (1.1) of the catheter device (1) into the sleeve (4), the method comprising the steps of:
providing the reducer (3) around the catheter device (1) proximally of the compressible part (1.1);
providing the sleeve (4) around the catheter device (1) proximally of the reducer (3);
providing the compression pipe (2) around the catheter device (1), such that the radially compressible part (1.1) is located between the movable fins (2.2);
and executing the following steps:
moving the reducer (3) towards the compression pipe (2), such that the compression pipe (2) slides into the distal opening of the reducer (3);
continuing the relative movement of the compression pipe (2) and the reducer (3) such that the movable fins (2.2) slide through the distal conical portion (3.2) of the reducer (3), effecting compression of the movable fins (2.2) from the open state to the closed state, thus compressing the radially compressible part (1.1);
continuing the relative movement such that the movable fins (2.2) in the compressed state and the compressed radially compressible part (1.1) slide along the proximal cylindrical portion (3.1); and
providing the sleeve (4) proximally against the compression pipe (2) or against a stop (3.14) pertaining to the reducer (3), the stop being provided proximally of the compression pipe (2), and pulling the catheter tube (1.3) in a proximal direction while holding on to the sleeve (4), such that at least the portion of the catheter device (1) comprising the radially compressible part (1.1) slides out of the compression pipe (2) and into the sleeve (4).

15. Method for using the system of claim 13 for inserting the radially compressible part (1.1) of the catheter device (1) into the sleeve (4), the method comprising the steps of:
providing the reducer (3) around the catheter device (1) proximally of the compressible part (1.1);
providing the sleeve (4) around the catheter device (1) proximally of the reducer (3);
providing the compression pipe (2) around the catheter device (1), such that the movable fins (2.2) face in the proximal direction;
and executing the following steps:
moving the reducer (3) and the disk device (3.4) towards the compression pipe (2), such that the disk device (3.4) pushes the compressible part (1.1) between the movable fins (2.2) and sliding the movable fins (2.2) into the distal opening of the reducer (3) until the disk device (3.4) gives in or breaks;
continuing the relative movement of the compression pipe (2) and the reducer (3) such that the movable fins (2.2) slide through the distal conical portion (3.2) of the reducer (3), effecting compression of the movable fins (2.2) from the open state to the closed state, thus compressing the radially compressible part (1.1);
continuing the relative movement such that the movable fins (2.2) in the compressed state and the compressed radially compressible part (1.1) slide along the proximal cylindrical portion (3.1); and
providing the sleeve (4) proximally against the compression pipe (2) or against a stop (3.14) pertaining the reducer (3), the stop being provided proximally of the compression pipe (2), and pulling the catheter tube (1.3) in a proximal direction while holding on to the sleeve (4), such that at least the portion of the catheter device (1) comprising the radially compressible part (1.1) slides out of the compression pipe (2) and into the sleeve (4).
